Europäisches Patentamt

(19) European Patent Office

Office européen des brevets

(11) Publication number : **0 440 387 A1**

(12) **EUROPEAN PATENT APPLICATION**

(21) Application number : **91300607.8**

(22) Date of filing : **28.01.91**

(51) Int. Cl.⁵ : **A61K 7/32**

(30) Priority : **30.01.90 GB 9002080**

(43) Date of publication of application :
**07.08.91 Bulletin 91/32**

(84) Designated Contracting States :
**AT BE CH DE DK ES FR GB GR IT LI NL SE**

(71) Applicant : **UNILEVER PLC**
**Unilever House Blackfriars P.O. Box 68**
**London EC4P 4BQ (GB)**
**GB**
Applicant : **UNILEVER NV**
**Burgemeester s'Jacobplein 1 P.O. Box 760**
**NL-3000 DK Rotterdam (NL)**
**BE CH DE DK ES FR GR IT LI NL SE AT**

(72) Inventor : **Park, Andrew Campbell**
**Quarry Road East**
**Bebington, Wirral, Merseyside L63 3JW (GB)**

(74) Representative : **Tonge, Robert James et al**
**UNILEVER PLC Patents Division P.O. Box 68**
**Unilever House**
**London EC4P 4BQ (GB)**

(54) Antiperspirant composition.

(57)    An antiperspirant composition which is suitable for topical application to human skin, comprising :
(i) from 4 to 95% by weight of hydrophobically-treated clay ; and
(ii) from 96 to 5% by weight of a carrier medium comprising an alkanol having from 1 to 4 carbon atoms ;
the composition containing not more than 0.2% by weight of aluminium.

EP 0 440 387 A1

# ANTIPERSPIRANT COMPOSITION

## FIELD OF INVENTION

The invention relates to antiperspirant compositions, particularly to products of the lotion type which are suitable for applying topically to the skin from a roll-on dispenser. The invention also relates to antiperspirant sticks, aerosols and dry powder products.

## BACKGROUND AND PRIOR ART

The appearance of perspiration at the skin surface, particularly in the underarm, has for many years exercised the ingenuity of cosmetic chemists in a search for products that can be applied topically to prevent, or at least reduce, the problems associated with sweating, notably the wetting of adjacent clothing and the developement of body malodour.

Conventional, highly effective antiperspirant products have traditionally been based on water soluble astringent aluminium salts as the active principle, which are capable of preventing the appearance of perspiration on the skin surface - if applied early enough - or at least of reducing the flow of perspiration.

Recent investigations into the safety in use of these astringent aluminium salts indicate that there may be some hazard to health in their repeated use topically, particularly as the concentration of aluminium ions in such products needs to be high if they are to be effective. By way of example, commercially available, highly effective underarm antiperspirant lotions contain up to 25% by weight of water soluble astringent aluminium salt, about one fifth of which is aluminium.

It is accordingly apparent that to be safe, the level of aluminium present in antiperspirant products of the conventional type should be very much less than the 5% that 25% by weight of this salt would contain, but this would render them virtually ineffective as antiperspirants.

While searching for alternative, safe yet effective, antiperspirant products for topical use, we have now discovered that the use of a relatively high level of a hydrophobically-treated clay, suspended in an alcoholic medium, can impart significant antiperspirancy, even when astringent metal salts are totally absent.

## DEFINITION OF THE INVENTION

Accordingly, the invention provides an antiperspirant composition which is suitable for topical application to human skin, comprising :

i) from 4 to 95% by weight of hydrophobically-treated clay, and

ii) from 96 to 5% by weight of a carrier medium comprising an alkanol having from 1 to 4 carbon atoms, or mixtures thereof ;

the composition containing not more than 0.2% by weight of soluble aluminium.

## DISCLOSURE OF THE INVENTION

### The Hydrophobically-Treated Clay

The composition according to the invention comprises a hydrophobically-treated clay or a mixture of such clays, in finely divided form, suspended, dispersed or dissolved in a carrier medium.

A preferred class of hydrophobically-treated clays comprises smectite clays, examples of which include the montmorillonites, hectorites, and colloidal magnesium aluminium silicates.

Montmorillonite is colloidal, hydrated aluminium silicate obtained from bentonite of which it is the predominant constituent. A detailed discussion of bentonites can be found in the Kirk-Othmer Encyclopedia of Chemical Technology, 2nd Edition, Vol. 3 (1964) pp. 339-360, published by Interscience Publishers, which is incorporated herein by reference.

Hectorite, also a smectite clay, differs from montmorillonite in that there is almost a complete substitution of aluminium in the lattice structure of montmorillonite by magnesium and in addition, it contains lithium and fluorine.

The magnesium aluminium silicates are complexes of colloidal magnesium aluminium silicate richer in magnesium than aluminium. The hydrophobically-treated magnesium aluminium silicates are commercially available under the name Veegum PRO from the R T Vanderbilt Co.

Preferred hydrophobically-treated clays for use in the present invention are those available under the trade name of "Bentone". Bentones are prepared by reacting suitable clays in a cation exchange system with an amine. Different amines are reacted to obtain a variety of Bentones, which may also differ in proportions of Si, MgO and $Al_2O_3$. Examples of useful "Bentone" suspending agents are "Bentone-27", which is a Stearalkonium hectorite ; "Bentone-34", which is Quaternium-18-bentonite ; "Bentone-38", which is Quaternium-18-hectorite; and "Bentone-14" which is a clay-extended Quaternium 18 hectorite, all of which have a particle size of below 5 microns and are commercially available from NL Industries Inc. Other suitable "Bentone" clays are Bentone SD1 and Bentone SD2.

Yet further suitable clays are hydrophobically-treated smectite clays available under the trade names Perchem and Tixogel. Specific examples are Perchem clays 44, 97 and 108 and Tixogel VZ.

Although hydrophobically-treated clays generally contain aluminium, the amount is extremely low. For example, the Bentones contain about 0.05% aluminium.

The hydrophobically-treated clay is preferably present in the composition in the form of deagglomerated platelet stacks. It should be explained that hydrophobically-treated clays as normally available from commercial suppliers are in the form of aggregates of platelet stacks and as such, sometimes do not function with maximum efficiency as antiperspirant actives in composition according to the invention. It is accordingly apparent that antiperspirant efficacy can be enhanced, if some degree of deagglomeration takes place before the composition according to the invention is finally formulated.

One convenient method for obtaining deagglomerated platelet stacks from the agglomerated form as is commercially available, is to subject the hydrophobically-treated clay to shearing in the presence of an alcohol or other polar liquid. An example of this shearing technique will be described later in this specification.

Alternatively, deaggregation can be achieved in the presence of an antiperspirant-inactive, water-soluble salt, such as sodium chloride.

The amount of hydrophobically-treated clay present in the composition according to the invention is from 4 to 95%, preferably from 5 to 50% and most preferably from 8 to 30% by weight.

If an amount of less than 4% by weight of the hydrophobically treated clay is employed, then the efficacy of the composition expressed in terms of sweat weight reduction (as hereinafter defined) can be so low as to be virtually undetectable.

A common feature of these hydrophobically treated clays is that the aluminium that they contain is in a water-insoluble form.

## The Carrier Medium

The composition according to the invention also comprises a carrier medium for the hydrophobically treated clay, which includes an alkanol having from 1 to 4 carbon atoms, or mixtures thereof. The preferred alkanol is chosen from ethanol, isopropanol and mixtures thereof.

The amount of the carrier medium present in the composition according to the invention is from 96 to 5%, preferably 95 to 50% and most preferably 92 to 70% by weight of the composition.

The carrier medium can accordingly consist essentially of the alkanol, as herein defined, or it can comprise other ingredients, particularly emollient liquids and adjuncts conventionally to be found in cosmetic products.

The carrier medium can be fluid as a free flowing liquid or a finely divided free-flow powder, or a solid, as a gel or cosmetic stick.

## Other Ingedients

The composition according to the invention can optionally also contain other ingredients in addition to the hydrophobically-treated clay and the alkanol.

## Emollient Liquid

Amongst optional ingredients, the composition according to the invention can comprise one or more liquid ingredients in addition to the alkanol, as herein defined. Such additional liquids can be incorporated to give emollient cosmetic benefits, and in particular can reduce skin irritation that can occasionally be encountered following contacting sensitive skin with an alkanol such as ethanol. The presence of an emollient liquid can also reduce or eliminate the white appearance of skin due to deposited clay particles after the alkanol has evaporated, that can follow topical application of the composition according to the invention.

The optional emollient liquid can be either a water-miscible hydrophilic liquid or a water-immiscible hydrophobic liquid.

Examples of suitable hydrophilic emollient liquids include polar liquids such as water-miscible polyoxyalkylene glycol or a water-miscible partial butyl ether thereof ; hexylene glycol ; a $C_1$-$C_4$ alkyl monoether of a simple or condensed $C_2$-$C_4$ alkylene glycol, for example dipropyleneglycol monomethyl ether ; and 2-ethyl-1,3-hexane diol.

Examples of other commercially available water-miscible hydrophilic emollient liquids are Pluronics (eg. Poloxamer 101, Poloxamer 105, Poloxamer 181, Poloxamer 182), Carbowaxes (eg. PEG-4, PEG-8, PEG-12), Witconol APEM (PPG-3 Myreth-3), Wiconol APES (PPG-9 Steareth-3), Standamul OXL (PPG-10 Ceteth-20), Procetyl AWS Modified (PPG-8 Ceteth-2), glycols and their citrate, lactate and tartrate esters. Particularly preferred hydrophilic emollient liquids which are especially useful are the dimethicone copolyols which are polymers of dimethylsiloxane with polyoxyethylene and/or polyoxypropylene side chains. Examples of these are SILWET L-720, L-7600, and L7610 from Union Carbide, Silicone 190 and 193 surfactants both from Dow Corning and ABIL B 8842, 8843 and 8851 from Goldschmidt. These dimethicone copolyols are also referred to in the literature as polyalkylene oxide modified dimethylpolysiloxanes, as silicone glycol copolymers and as polysilicone polyether copolymers. Dimethyl isosorbide is a further example of a suitable hydrophilic emollient liquid.

The amount of water-miscible hydrophilic emollient liquid that optionally can be present in composition according to the invention is generally from 0 to 20%, preferably from 1 to 15% by weight.

When a water-immiscible hydrophobic liquid is present in the composition, the amount employed will depend upon whether it is less volatile or more volatile than ethanol.

Examples of water-immiscible hydrophobic emollient liquids which are less volatile than ethanol that may be present in minor proportion are non-polar liquids such as the linear volatile silicones having 2 to 9 silicone atoms such as hexamethyldisiloxane, the cyclic volatile silicones having 3 to 6 silicone atoms such as the tetramer and pentamer and mixtures thereof, paraffin oils, fatty acid esters such as isopropyl myristate and dibutyl phthalate, and polyoxypropylene fatty ethers such as Fluid AP.

The amount of water-immiscible hydrophobic emollient liquid, less volatile than ethanol, which optionally can be present in the composition according to the invention is generally from 0 to 5%, preferably from 0.5 to 3% by weight.

Examples of water-immiscible hydrophobic emollient liquids which are more volatile than ethanol that may be present in the composition, are non-polar liquids such as certain liquefiable gaseous propellants. Examples of propellants are given below.

The amount of water-immiscible emollient liquid more volatile than ethanol which optionally can be present in the composition according to the invention is generally from 0 to 80% by weight.

## Perfume

The composition according to the invention can also optionally comprise a perfume. If present, then it should be chosen from any sutiable perfume that is compatible with the other ingredients present in the final composition. A particularly preferred perfume is a deodorant perfume, such as one described in GB 2 013 493 (Unilever Limited).

The amount of perfume when employed in the composition according to the invention is preferably from 0.001 to 2%, most preferably from 0.1 to 1% by weight.

## Liquefiable Propellant Gas

The composition according to the invention can also optionally comprise a liquefiable propellant gas, particularly when the composition is intended to be dispensed as a spray from an aerosol container.

Examples of liquefiable propellant gases that can be employed in accordance with the invention are non-halogenated hydrocarbons, fluorocarbons, chlorofluorocarbons, bromofluorocarbons, methylene chloride, dimethyl ether and mixtures thereof.

Specific examples of non-halogenated hydrocarbons are propane, butane, 2-methylpropane and mixtures thereof.

Specific examples of fluorocarbons are octafluorocyclobutane (Propellant C-318), monofluoroethane (Propellant 1141), difluoroethane (Propellant 152a), tetrafluoroethane (Propellant-134a) and mixture thereof.

Specific examples of chlorofluorocarbons are
dichlorofluoromethane (Propellant 21),
dichlorotetrafluoroethane (Propellant 114),
chlorodifluoromethane (Propellant 22),
chloropentafluoroethane (Propellant 115),
dichlorodifluoromethane (Propellant 12),
chlorodifluoroethane (Propellant 142b),

trichlorofluoromethane (Propellant 11),
trichlorofluoroethane (Propellant 113), and mixtures thereof.

A specific example of a bromofluorocarbon is bromotrifluoromethane.

The amount of liquefiable propellant gas that can be employed is that which is conventional in the art.

## Water-absorbent polymer

The composition according to the invention can also optionally comprise a water-absorbent polymer that can serve to absorb moisture, particularly perspiration, at the skin surface following topical application of the composition.

Preferably, the water-absorbent polymer should be one which is in the form of a finely divided powder which is dry-to-the-touch and not sticky in use. The polymer should also preferably possess the ability to absorb an amount of moisture at least equal to its own weight, most preferably at least equal to ten times its own weight following topical application of the composition.

Examples of suitable polymers include :

Chemically modified starches, such as FOXORB 15,

cross-linked etherified starch as described in US 4 508 705

cross-linked gelatinised starch as described in US 4 508 705

Other moisture absorbent polymers as described in US 4 743 440.

Chemically modified celluloses, such as

AKUCELL SW 009

AQUASORB,

AQUALON, and

cross-linked sodium carboxymethyl cellulose as desbribed in US 3 965 091.

Starch-acrylonitrite graft copolymer as described in US 4 508 705

Poly-acrylate cross-linked with polyamide/epichlorhydrin material as described in DE 2 614 602.

Other anionic polyelectrolytes as described in US 4 508 705.

Polyhipes and modified Polyhipes, such as sodium sulphonated Polyhipes as described in EP-A-0 060 138

The amount of water-absorbent polymer, when employed in the composition according to the invention, can be from 0.1 to 50%, preferably from 1 to 25% by weight of the composition.

## Gelling Agent

The composition according to the invention can also optionally comprise a gelling agent, particularly when it is intended to take the form of a solid cosmetic stick. The gelling agent can accordingly be employed to function as a structural matrix. In general, preferred gelling agents have a melting point of from 45°C to 75°C. Examples of gelling agents include fatty acids, fatty alcohols, fatty acid esters and fatty acid amides, each having from 8 to 30 carbon atoms in the molecule. Particularly preferred examples of gelling agents are cetyl alcohol, palmitic acid, isopropyl palmitate, stearyl alcohol, eicosanyl alcohol, behenyl alcohol, lauryl alcohol, behenamide, the sucrose esters of tallow fatty acids, the mon- and di- fatty acid esters of polyethylene glycol and dibenzaldehyde monosorbitol acetal.

It should be noted that if dibenzaldehyde monosorbitol acetal is employed as the gelling agent, then it is also desirable to include a solubilising agent therefor, examples of which include 1,2-propyleneglycol, hexyleneglycol and polyethylene glycol.

The amount of gelling agent when employed in the composition according to the invention can be from 0.5 to 30%, preferably from 1 to 25% by weight of the compositions.

If an amount of less than 0.5% by weight of the gelling agent is employed, then the composition is likely to be too soft in use. Also, if an amount in excess of 30% by weight of the gelling agent is employed, then the product is likely to be too hard in use.

## Other antiperspirant adjuncts

The composition according to this invention can also optionally comprise antiperspirant adjuncts other than the materials already referred to in the specification, in order to enhance or improve the properties of the final composition. Examples of other antiperspirant adjuncts can include colourants and antimicrobial agents, such as 2,4,4' ,-trichloro-2' ,-hydroxydiphenyl ether (IRGASAN DP 300).

Product Form and Container

The compositions of the invention can be formulated as liquids, for example as a lotion or flowable gel for use in conjunction with an applicator such as a roll-ball applicator, or a spray device such as an aerosol can containing propellant, or a container fitted with a pump to dispense the liquid product. Alternatively, the compositions of the invention can be solid or semi-solid, for example sticks, creams or firm gels, for use in conjunction with a suitable applicator, or simply a tube, bottle or lidded jar, or as a liquid-impregnated fabric, such as a tissue wipe.

The invention accordingly also provides a closed container containing a composition as herein defined.

Use of The Composition

The invention also provides for the use of the antiperspirant composition, as herein defined, for topical application to mammalian skin.

The compositions according to the invention are primarily intended particularly for topical application to the underarm of the human subject, or to other parts of the body where perspiration occurs. The composition can according be applied as frequently as desired and in amount that are sufficient to reduce or eliminate perspiration.

PROCESS FOR MANUFACTURING THE ANTIPERSPIRANT PRODUCT

The invention also relates to a process for the manufacture of the antiperspirant composition which comprises the step of shearing the hydrophobically-treated clay in at least part of the alkanol, until a reading of at least 5 is obtained on a Brookfield RTV 100 Viscometer using the A spindle at 20 rpm in the helical mode, at 20°C. This reading corresponds to a viscosity of about 500 mPas.

This shearing step is intended to deagglomerate (ie to break down) the normally agglomerated particles of hydrophobically-treated clay, so as to provide individual platelets or platelet stacks of the clay, since it is apparent that antiperspirant efficacy of the composition is thereby enhanced.

The product so obtained is finally filled into a suitable container and sealed prior to sale and use, to prevent evaporative loss of the more volatile ingredients.

MEASURING THE EFFICACY OF THE ANTIPERSPIRANT PRODUCT

In the following part of this specification references are made to the antiperspirant efficacy of various products. Before giving details of the composition of these products the test procedure carried out to evaluate their antiperspirant efficacy will first be described. The test procedure involves subjecting human volunteers to thermal stress and gravimetric determination of axilla sweat.

Subjects :

A panel of up to 60 women who use no antiperspirant for the 14 days before the test.

Hot Room :

Temperatures 40°C ± 2°C ; relative humidity 40% ± 5%.

Products :

When testing two products one being designated the test product and the other the control, the panel is divided into two equal groups. One group receives the test treatment on the left axilla and the control treatment on the right, while the second group receives them the other way round.

Control Product :

This is a placebo deodorant in the form of an aerosol product comprising 25% ethanol, 0.6% ispropyl myristate, 0.3% perfume, and 74.1% propellant (1 :1 mixture of Propellants 11 & 12).

Product Application :

The operator conducting the test applies the test product in the standard manner so as to deposit an appropriate quantity of product, for example, on average about 300 mg of roll-on product on each axilla.

Sweat Collection :

Absorbent cotton pads are used to collect the sweat. On entering the hot room each panellist is subjected to a 40 minute 'warm-up' period, during which no sweat is collected. Sweat is then collected for a 20 minute period and sweat weight determined.

Test Design :

Subjects attend daily for 3 consecutive days. They receive one treatment with the products on each of the first three days. Following product application on the third day, the panellist is subjected to a hot room sitting and sweat is collected.

Analysis of Data :

The statistical treatment includes an analaysis of variance which allows for side effects due to the product and the panellist. The efficacy is calculated from the geometric mean weight of sweat collected from the axillae treated with each product using the formula :

$$\% \text{ reduction} = 100 \frac{(C-T)}{C}$$

where C is the geometric mean sweat weight from the axillae treated with the control product and T is the geometric mean sweat weight from the axillae treated with the test product where a correction has been made for the side effect.

Significance is calculated by applying Student's t-test to the logarithmically transformed weights.

### EXAMPLES

The invention is illustrated by reference to the following examples

### Example 1

This example illustrates an antiperspirant roll-on lotion (A) according to the invention, emphasising the benefit of shearing of the ingredients in ethanol to deagglomerate the hydrophobically-treated clay.

In order to prepare this Lotion (A), Bentone 38 was sheared in the ethanol using an Ultral Turrax mixer fitted with a 45N shaft and a T45/6G generator until a reading of at least 5 was obtained with a Brookfield RTV100 Viscometer using the A spindle at 20 rpm in the helical mode, at 20°C. Perfume was then added to complete preparation of the roll-on lotion.

By way of comparison, a control Lotion (B) was prepared by simple mixing of the identical ingredients, but without shearing.

Both Lotions contained the following ingredients :

|  | %/w |
|---|---|
| Bentone 38 | 10 |
| Ethanol | 89 |
| Perfume | 1 |

The ability of Lotions A & B to reduce perspiration was then tested on a panel of female volunteers according to the method described hereinbefore, and the following results were obtained.

|                          | Efficacy expressed as % sweat weight reduction |
|--------------------------|:----:|
| Lotion A (Sheared)       | 20   |
| Lotion B (Unsheared)     | 4    |

These results confirmed that shearing of Bentone 38 in aqueous ethanol considerably improves the antiperspirant efficacy of the roll-on lotion.

Example 2

This example illustrates further antiperspirant Lotions (C & D) according to the invention, emphasising the benefit of salt to deagglomerate the hydrophobically-treated clay.

In order to prepare Lotion (C), Bentone 38 was sheared in ethanol, as described in Example 1, and then salt and perfume were added with simple mixing.

By way of comparison, a further Lotion (D) was prepared by simple mixing of identical ingredients, but without shearing.

Both lotions contain the following ingredients :

|                    | % w/w |
|--------------------|:-----:|
| Bentone 38         | 10    |
| Ethanol            | 64    |
| Sodium chloride    | 25    |
| Perfume            | 1     |

The ability of Lotions C & D to reduce perspiration was then tested on a panel of female volunteers according to the method disclosed hereinbefore, and the following results were obtained :

|                          | Efficacy expressed as % sweat-weight reduction |
|--------------------------|:----:|
| Lotion C (Sheared)       | 14   |
| Lotion D (Unsheared)     | 13   |

These results confirm that shearing of Bentone 38 in aqueous ethanol does not significantly further enhance the antiperspirant efficacy in the presence of a substantial amount of salt, it being apparent that the salt alone will deagglomerate the Bentone 38 without shearing (Lotion D) to provide a roll-on lotion having better antiperspirant efficacy, than a similar unsheared lotion (see Lotion B of Example 1) which is free from salt.

Example 3

This example illustrates a further antiperspirant Lotion (E) containing a satisfactory amount of an optional water-miscible emollient, namely, dipropylene glycol methyl ether. By way of comparison a further Lotion (F) containing an equivalent amount of a water-immiscible emollient which is less volatile than ethanol, namely Volatile silicone 7207, is included to demonstrate the importance of restricting the amount of such an emollient to avoid loss of antiperspirant efficacy.

8

The Lotions contained the following ingredients :

|  | % w/w | |
|---|---|---|
|  | Lotion E | Lotion F |
| Bentone 38 | 10 | 10 |
| Ethanol | 74 | 74 |
| Dipropylene glycol methyl ether | 15 | -- |
| Volatile Silicone 7207 | -- | 15 |
| Perfume | 1 | 1 |

The ability of Lotions E & F to reduce perspiration was then tested on a panel of female volunters according to the method disclosed hereinbefore, and the following results were obtained :

|  | Efficacy expressed as % sweat-weight reduction |
|---|---|
| Lotion E (sheared) | 9 |
| Lotion F (sheared) | 0 |

These results confirm that the presence in the composition of an optional water-miscible emollient (Lotion E) at a concentration of 15% by weight, within the stipulated range of up to 20% by weight provides an acceptable level of efficacy. In contrast, the presence in the composition of an optional water-immiscible emollient (Lotion F) at a concentration of 15%, i.e. in excess of the stipulated range of up to 5% by weight, completely negates the antiperspirant efficacy of the composition.

## Example 4

This example demonstrates two further formulations which were found to be particularly effective in sweat weight reduction, when tested by the method herein before described.

|  | % w/w | |
|---|---|---|
|  | Lotion G | Aerosol Formulation H |
| Akucell SW3009 | 10.0 | 2.0 |
| Zinc Carbonate | 3.0 | 0.5 |
| Bentone 38 | 10.0 | 2.0 |
| Ethanol | 76.0 | 10.0 |
| Perfume | 1.0 | 1.0 |
| n-Pentane | – | 40.0 |
| Cap 70 (a hydrocarbon) | – | 44.5 |

Both formulations were prepared by a similar method to that described in Example 1, except that in the case of aerosol formulation H all of the ingredients except the Cap 70 were sheared or simply mixed together, the Cap 70 (a propellant gas) being added when the formulation was dosed into the can, by known techniques.

When tested on a panel of female volunteers according to the method disclosed hereinbefore, the following results were obtained.

| | Efficacy expressed as % sweat-weight reduction |
|---|---|
| Lotion G (Sheared) | 28 |
| Lotion H (Unsheared) | 24 |

## Claims

1. An antiperspirant composition which is suitable for topical application to human skin, comprising :
   (i) from 4 to 95% by weight of hydrophobically-treated clay ; and
   (ii) from 96 to 5% by weight of a carrier medium comprising an alkanol having from 1 to 4 carbon atoms;
   the composition containing not more than 0.2% by weight of aluminium.

2. A composition according to claim 1, in which the hydrophobically-treated clay is a smectite clay.

3. A composition according to claim 2, in which the smectite clay is a montmorillonite clay.

4. A composition according to claim 3, in which the montmorillonite clay is Quaternium-18-bentonite.

5. A composition according to claim 2, in which the smectite is a hectorite.

6. A composition according to claim 5, in which the hectorite is Stearalkonium hectorite.

7. A composition according to claim 5, in which the hectorite is Quaternium-18-hectorite.

8. A composition according to claim 2, in which the smectite clay is colloidal magnesium aluminium silicate.

9. A composition according to claim 2, in which the smectite clay is Bentone SD-1.

10. A composition according to any preceding claim, in which the hydrophobically-treated clay comprises deagglomerated platlet stacks.

11. A composition according to any preceding claim, in which the hydrophobically-treated clay forms from 5 to 50% by weight of the composition.

12. A composition according to claim 11, in which the hydrophobically-treated clay forms from 8 to 30% by weight of the composition.

13. A composition according to any preceding claim in which the alkanol is chosen from ethanol, isopropanol and mixtures thereof.

14. A composition according to any preceding claim, in which the carrier medium forms from 95 to 50% by weight of the composition.

15. A composition according to claim 14, in which the carrier medium forms from 92 to 70% by weight of the composition.

16. A composition according to claim any preceding claim, further comprising a water-absorbent polymer which possesses the ability to absorb an amount of water at least equal to its own weight.

17. A composition according to claim 16, in which the water-absorbent polymer possesses the ability to absorb an amount of water at least equal to 10 times its own weight.

18. A composition according to claim 15, 16 or 17, in which the water-absorbent polymer comprises a chemically modified cellulose.

19. A composition according to claim 15, 16 or 17, in which the water-absorbent polymer comprises a chemically modified starch.

20. A composition according to any of claims 16 to 19, in which the water-absorbent polymer forms from 0.1 to 50% by weight of the composition.

European Patent Office

**EUROPEAN SEARCH REPORT**

Application Number

EP 91 30 0607

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | FR-A-2 269 923 (THE PROCTER & GAMBLE CO.)<br>* Page 2, lines 26-37; page 3, line 13 - page 4, line 22; page 6, lines 26-29; claims 1-5 *<br>--- | 1-8,13 | A 61 K 7/32 |
| Y | FR-A-2 274 278 (COLGATE PALMOLIVE CO.)<br>* Page 3, line 30 - page 5, line 7; claims 1-5 * | 1-5 | |
| A | | 6-8 | |
| | --- | | |
| Y | EP-A-0 327 382 (ECC INTERNATIONAL LTD)<br>* Claims 1-4,12 * | 1-5 | |
| A | | 13,16, 18 | |
| | --- | | |
| Y | GB-A-2 082 614 (NATIONAL STARCH & CHEM. CORP.)<br>* Page 1, line 47 - page 4, line 46 * | 1-5 | |
| A | | 16,18, 19 | TECHNICAL FIELDS SEARCHED (Int. Cl.5) |
| | ----- | | A 61 K |

The present search report has been drawn up for all claims

| Place of search | Date of completion of the search | Examiner |
|---|---|---|
| THE HAGUE | 26-04-1991 | BERTOCCHI C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)